# EUROPÄISCHE PATENTSCHRIFT

(11) **EP 2 298 256 B1**
(45) Veröffentlichungstag und Bekanntmachung des Hinweises auf die Patenterteilung: **03.10.2012**
(21) Anmeldenummer: 10009126.3
(22) Anmeldetag: 07.09.2006
(51) Int. Cl.: A61F 9/01

(54) **Ophthalmologische Vorrichtung für die refraktive Korrektur eines Auges**
Ophthalmological device for the refractive correction of an eye
Dispositif ophtalmologique pour la correction réfractive de l'oeil

(43) Veröffentlichungstag der Anmeldung: 23.03.2011
(62) Teilanmeldung aus: 06405385.3
(73) Patentinhaber: Ziemer Holding AG, 2562 Port (CH)
(72) Erfinder: Rathjen, Christian, 28197 Bremen (DE)
(74) Vertreter: Rentsch Partner AG

(56) Entgegenhaltungen:
- EP-A2- 1 627 617
- EP-B1- 1 731 120
- US-A1- 2003 212 387
- US-B1- 6 325 792
- Alexander Heisterkamp: "Einsatz ultrakurzer Laserpulse in der refraktiven Laserchirurgie", Deutsche Nationalbibliothek , 2002, Seiten 75-77, XP002638596, Deutsche Nationalbibliothek Gefunden im Internet: URL:http://deposit.ddb.de/cgi-bin/dokserv? idn=966097009&dok_var=d1&dok_ext=pdf&filen ame=966097009.pdf [gefunden am 2011-05-24]

## Beschreibung

### Technisches Gebiet

Die vorliegende Erfindung betrifft eine ophthalmologische Vorrichtung für die refraktive Korrektur eines Auges. Die Erfindung betrifft insbesondere eine ophthalmologische Vorrichtung für die refraktive Korrektur eines Auges mittels Projektion von Laserpulsen auf einen Brennpunkt im Innern des Auges für eine Auflösung von Augengewebe.

### Stand der Technik

Fehlsichtigkeiten wie Myopie (Kurzsichtigkeit), Hyperopie (Weitsichtigkeit oder Übersichtigkeit) oder Astigmatismus (Stabsichtigkeit) können heute durch refraktiv-chirurgische Behandlung dauerhaft korrigiert werden. Refraktiv-chirurgische Behandlungen sind chirurgische Eingriffe am Auge, die die optische Brechkraft des Auges ändern mit dem Ziel, diese einem gewünschten Wert möglichst gut anzunähern. Mittels Femtolasersystemen, die Pulsbreiten von typisch 10fs bis 1000fs (1fs=10⁻¹⁵s) aufweisen, können transparente Materialen im Fokus durch nichtlineare Absorption und anschliessende Wechselwirkung (z.B. Photodisruption) bearbeitet werden. Insbesondere werden in der Praxis in der Augenhornhaut (Cornea) durch Gewebeauflösung mittels Femtolaserpulsen operativ Schnitte erzeugt.

In der Patentschrift US 5,993,438 wird ein Verfahren für die refraktive Keratektomie in der Augenhornhaut mittels gepulster Laserstrahlen beschrieben. Gemäss US 5,993,438 wird durch Gewebeauflösung im Innern der Augenhornhaut ein domförmiger Hohlraum um die optische Achse erzeugt, welcher beim Kollabieren die Hornhautkrümmung geeignet verändert. Der zusammenhängende Hohlraum wird durch mehrere direkt übereinander liegende Abtragungsschichten gebildet, die zentralsymmetrisch um die optische Achse des Auges angeordnet sind. Jede der Abtragungsschichten wird durch beispielsweise spiralförmig hintereinander gereihte Laserpulse zentralsymmetrisch um die optische Achse herum erzeugt. Mit abnehmender Distanz zu der Hornhautoberfläche weisen die Abtragungsschichten jeweils einen abnehmenden Durchmesser auf. Die Distanz zwischen den Fokusdurchmessern von hintereinander folgenden Laserpulsen beträgt nach US 5,993,438 vorzugsweise das Ein- bis Zweifache des Radius einer durch einen der Laserpulse im Brennpunkt erzeugten Blase. In einer Abtragungsschicht ist das Augengewebe jeweils über eine Dicke von ungefähr 10µm aufgelöst. Die übereinander liegenden Abtragungsschichten werden jeweils direkt aneinandergrenzend erzeugt, so dass der domförmige Hohlraum zusammenhängend, ohne verbleibende Gewebebrücken gebildet wird. Das Verfahren nach US 5,993,438 eignet sich zwar für die Behandlung von Myopie, der domförmige Hohlraum ist jedoch nicht für die Korrektur von Hyperopie, Astigmatismus oder Aberrationen (Abbildungsfehler) höherer Ordnung geeignet.

EP 1 731 120 beschreibt eine ophthalmologische Vorrichtung mit einem Polygonscanner, der eine Rotations- oder Ablenkgeschwindigkeit von 100-1000Hz aufweist, was einer Ablenkungsgeschwindigkeit von 1-10m/s entspricht. Die Vorrichtung nach EP 1 731 120 weist überdies einen optionalen weiteren Scanner auf, der die von der Lichtquelle abgegebenen Lichtpulse mittels eines weiteren rotierenden Spiegels senkrecht zur Abtastrichtung ablenkt. Überdies ist ein Rotationselement vorgesehen, beispielsweise ein um eine optische Achse drehbarer K-Spiegel, welches ermöglicht, die Abtastebene, in welcher die in die Abtastrichtung abgelenkten Lichtpulse verlaufen, um die optische Achse zu drehen.

US 2003/0212387 beschreibt ein Lasersystem für die Materialbearbeitung, z.B. zur refraktiven Korrektur eines Auges, das während der Behandlung in einer statischen Lage fixiert wird, mit X, Y, Z-Achsen Galvanoscannern und einem Computer System zum Steuern der Bewegung des Laserstrahls entlang einem selektierten definierten Pfad.

US 6,325,792 beschreibt ein ophthalmologisches Lasersystem mit einem auf einen Strahlsteuerungs-Scanner, das ein Eyetracker System einsetzt, welches ausgehend von einer Anfangsreferenzposition jegliche Augenbewegungen überwacht und zur Kompensation die Position des Laserstrahls entsprechend anpasst.

EP 1 627 617 beschreibt eine ophthalmologische Vorrichtung für die Auflösung von Augengewebe, welche die translatorische Bewegung eines Brennpunkts durch mechanisches Bewegen eines Lichtprojektors ausführt. Als Alternative werden für die Bewegung des Brennpunkts in der y-Richtung bewegliche lichtumlenkende optische Elemente erwähnt, welche die Bewegung des Brennpunkts schneller ausführen als die mechanische Bewegung, beispielsweise ein Scannen in der y-Richtung mit einer Frequenz von 100-1000Hz. Die Vorrichtung nach EP 1 627 617 ermöglicht überdies die Überlagerung einer feinen Bewegung des Brennpunkts mittels optischen Microscans auf die translatorischen Bewegungen in der x-Richtung und y-Richtung. Zum Fixieren der Vorrichtung am Auge ist ein Saugring vorgesehen.

Im Artikel von Alexander Heisterkamp: "Einsatz ultrakurzer Laserpulse in der refraktiven Laserchirurgie", Deutsche Nationalbibliothek, 2002, Seiten 75-77, wird ein Verfahren zur Augenbehandlung beschrieben, in welchem das Auge über eine Saugringeinheit fixiert wird und ein X-Y-Galvanometer-Scannersystem mit auf Achsen montierten Galvanometerspiegeln zur Strahlablenkung verwendet wird.

### Darstellung der Erfindung

Es ist eine Aufgabe der vorliegenden Erfindung, eine neue ophthalmologische Vorrichtung für die refraktive Korrektur eines Auges mittels Laserpulsen vorzuschlagen, welche insbesondere nicht auf die Korrektur der Kurzsichtigkeit beschränkt sind.

Gemäss der vorliegenden Erfindung werden diese Ziele durch die Elemente des unabhängigen Anspruchs erreicht. Weitere vorteilhafte Ausführungsformen gehen ausserdem aus den abhängigen Ansprüchen und der Beschreibung hervor.

Die oben genannten Ziele werden durch die vorliegende Erfindung insbesondere dadurch erreicht, dass die ophthalmologische Vorrichtung, welche einen Lichtprojektor umfasst zur Projektion von Laserpulsen auf einen Brennpunkt im Innern des Auges zur Auflösung von Augengewebe, zudem ein Positionierungsmodul und ein Abtastmodul umfasst. Das Positionierungsmodul ist eingerichtet, den Brennpunkt an unterschiedliche Ausgangspunkte zu positionieren. Das Abtastmodul ist eingerichtet, den Brennpunkt ausgehend von jeweils einem der Ausgangspunkte gemäss einem Abtastmuster für ein Bearbeitungsteilgebiet zu bewegen, wobei das Abtastmuster und die Ausgangspunkte so definiert sind, dass das Augengewebe in mehreren durch Gewebebrücken voneinander getrennten Bearbeitungsteilgebieten aufgelöst wird. Das Abtastmuster definiert beispielsweise ein Bearbeitungsteilgebiet mit einer rechteckigen, runden, elliptischen, sternförmigen oder spiralförmigen Form oder in einer Form ähnlich einer Lissajou-Figur. Zum Ablenken der Laserpulse umfasst das Abtastmodul beispielsweise einen Galvanoscanner, einen resonanten Spiegelscanner, einen akustischen optischen Modulator, einen Polygonscanner und/oder einen mikroelektromechanischen Scanner. Das Positionierungsmodul umfasst Bewegungstreiber zum mechanischen Verschieben von mindestens Teilen des Lichtprojektors und/oder einen Galvanoscanner zum Ablenken der Laserpulse. Der Lichtprojektor weist vorzugsweise eine numerische Apertur von über 0.3 auf. Die ophthalmologische Vorrichtung umfasst beispielsweise ein Steuermodul, welches eingerichtet ist, das Positionierungsmodul und das Abtastmodul so zu steuern, dass das Positionierungsmodul den Brennpunkt so an unterschiedliche Ausgangspunkte positioniert und dass das Abtastmodul den Brennpunkt ausgehend von jeweils einem der Ausgangspunkte gemäss dem Abtastmuster so bewegt, dass das Augengewebe in mehreren durch Gewebebrücken voneinander getrennten Bearbeitungsteilgebieten aufgelöst wird. Durch die Bildung einer Vielzahl von getrennten, nicht zusammenhängenden Bearbeitungsteilgebieten mit aufgelöstem Augengewebe ist es möglich die Krümmung der Augenhornhaut nicht bloss wie im Stand der Technik zentralsymmetrisch zur Korrektur einer Myopie abzuflachen, sondern für eine refraktive Korrektur die Krümmung der Augenhornhaut an beinahe beliebigen Stellen und insbesondere auch asymmetrisch zu ändern. Beispielsweise können, durch geeignete Wahl der Ausgangspunkte, mehrere der Bearbeitungsteilgebiete in einem ringförmigen Cluster im (intrastromalen) Hornhautgewebe so angeordnet werden, dass eine Hyperopie korrigiert werden kann. Durch unterschiedliche Verteilung der Bearbeitungsteilgebiete in der Hornhaut, sowohl in der Tiefe als auch in der Distanz zur optischen Achse des Auges, und/oder durch mehrschichtige Anordnung der Bearbeitungsteilgebiete im Hornhautgewebe kann die Hornhautkrümmung zur Korrektur von Astigmatismus und Aberrationen höherer Ordnung geeignet verändert werden. Neben der Behandlung und Korrektur der Augenhornhaut ist es zudem auch möglich, durch die ophthalmologische Vorrichtung auf die gleiche Art das Gewebe der Augenlinse zu behandeln, insbesondere zur Verbesserung der Elastizität der Linse bei Altersweitsichtigkeit.

In einer bevorzugten Ausführungsvariante ist das Positionierungsmodul eingerichtet, den Brennpunkt jeweils an Ausgangspunkte auf einer ersten Bearbeitungsfläche zu positionieren, und das Abtastmodul ist eingerichtet, den Brennpunkt in dieser ersten Bearbeitungsfläche zu bewegen. Die ophthalmologische Vorrichtung umfasst zudem ein Tiefenpositionierungsmodul zum Verschieben des Brennpunkts entlang einer Projektionsachse des Lichtprojektors in eine zur ersten Bearbeitungsfläche äquidistante, z.B. parallele, zweite Bearbeitungsfläche, so dass der Brennpunkt in der zweiten Bearbeitungsfläche an unterschiedliche Ausgangspunkte positionierbar und ausgehend von jeweils einem der Ausgangspunkte gemäss dem Abtastmuster bewegbar ist. Durch die Tiefenpositionierung des Brennpunkts können mehrere Fokalflächen, z.B. Fokalebenen, eingestellt werden, welche jeweils eine Bearbeitungsfläche, z.B. Bearbeitungsebene, bilden, auf der die Ausgangspunkte jeweils definiert und das Augengewebe in Bearbeitungsteilgebieten aufgelöst wird. Die Tiefenpositionierung des Brennpunkts ermöglicht somit eine mehrschichtige Bearbeitung des Augengewebes mit jeweils einer Vielzahl von getrennten, nicht zusammenhängenden Bearbeitungsteilgebieten, in denen das Augengewebe aufgelöst wird. Vorzugsweise wird dabei die Distanz zwischen einzelnen Fokalflächen respektive Bearbeitungsflächen so bestimmt, dass bei übereinander liegenden Bearbeitungsteilgebieten benachbarter Bearbeitungsflächen, jeweils eine Gewebebrücke bestehen bleibt. Zu diesem Zweck ist das Steuermodul zudem vorzugsweise eingerichtet, das Tiefenpositionierungsmodul so zu steuern, dass beim Verschieben des Brennpunkts eine Mindestdistanz zwischen den Bearbeitungsflächen eingehalten wird, wobei die Mindestdistanz so definiert ist, dass in äquidistanten (parallelen) Bearbeitungsflächen über einander liegende Bearbeitungsteilgebiete durch Gewebebrücken voneinander getrennt sind. Der Zweck und Vorteil der Gewebebrücken besteht darin, dass definierte Abtragschichtdicken erzeugt werden können. Es hat sich nämlich herausgestellt, dass bei der Laserbearbeitung entstehende innere Gasdrucke eine Deformation des Gewebes erzeugen, die die Präzision beim schichtweisen Abtragen von grossen zusammenhängenden Schichten, wie es im Stand der Technik beschrieben wird, stark beeinträchtigt.

In einer Ausführungsvariante ist das Steuermodul eingerichtet, entsprechend einer gewünschten refraktiven Korrektur des Auges, die Anzahl der Bearbeitungsteilgebiete sowie die Ausgangspunkte für die örtliche Verteilung der Bearbeitungsteilgebiete in mehreren Bearbeitungsflächen im Innern des Auges zu bestimmen. Das Steuermodul bestimmt die örtliche Verteilung der Bearbeitungsteilgebiete beispielsweise auf Grund eines Modells des zu behandelnden Augengewebes, z.B. ein Hornhautmodel, bei vorgegebener Grösse und Form der Bearbeitungsteilgebiete und bei vorgegebenen vertikalen und horizontalen Mindestabständen einzelner Bearbeitungsteilgebiete.

In einer Ausführungsvariante ist das Steuermodul zudem eingerichtet, unterschiedliche Abtastmuster für unterschiedlich grosse Bearbeitungsteilgebiete zu wählen.

In einer bevorzugten Ausführungsvariante umfasst die Vorrichtung einen Wellenfrontdetektor zum Bestimmen eines Wellenfrontverlaufs eines durch das Auge reflektierten Lichtbündels. Das Steuermodul ist zudem eingerichtet, die Ausgangspunkte basierend auf dem bestimmten Wellenfrontverlauf festzulegen. Das heisst, das Steuermodul ist eingerichtet, die örtliche Verteilung der Bearbeitungsteilgebiete basierend auf dem bestimmten Wellenfrontverlauf festzulegen. Dadurch kann die erreichte refraktive Korrektur während der Behandlung gemessen werden und, darauf basierend, die Positionierung weiterer Bearbeitungsteilgebiete, so weit nötig, bestimmt werden.

Vorzugsweise ist das Abtastmodul eingerichtet, nacheinander folgende Laserpulse so zu positionieren, dass sich ihre Fokusdurchmesser teilweise überlappen. Vorzugsweise überlappen sich ihre Fokusdurchmesser mindestens bis zur Hälfte ihres Durchmessers. Durch die Überlappung der Fokusdurchmesser können Laserpulse mit geringere Pulsenergie für die Gewebeauflösung eingesetzt werden, wodurch nur geringe mechanische Spannungen durch Gas und Kavitationsblasen im Restgewebe induziert werden, was bei der Bildung von gleichmässig dünnen Abtragsbereichen hilft und einer definierten Kollabierung der Bearbeitungsteilgebiete förderlich ist. Insbesondere zusammen mit der Verwendung hoher numerischer Aperturen, beispielsweise >0.3, insbesondere >0.4, und der damit verbundenen geringeren erforderlichen Pulsenergien, lassen sich sehr regelmässige Abtragsvolumina mit geringer Höhe und geringem Aspektverhältnis erzeugen (Annäherung an Sphäre). Es ist sogar möglich mit hoher numerischer Apertur und Pulsen von sehr kurzer Dauer und geringer Energie gasarme oder sogar gasfreie Schnitte zu erzeugen. Überhitzungen, wie im Stand der Technik erwähnt, treten dabei selbst bei grossen Überlappungen einzelner Laserpulse nicht auf.

Vorzugsweise ist das Abtastmodul eingerichtet, den Brennpunkt wesentlich schneller zu bewegen als die Bewegungsgeschwindigkeit eines menschlichen Auges bei einer Blickrichtungsänderung. Wenn das Auge während der Behandlung nicht mechanisch fixiert wird und sich bewegt, dann kann zwar die Grösse des durch das Abtastmuster definierten Bearbeitungsteilgebiets durch die Augenbewegung leicht verändert werden, doch das Abtastmodul bewegt den Brennpunkt schnell genug, um zu verhindern, dass im Bearbeitungsteilgebiet auf Grund der Augenbewegungen Gewebebrücken verbleiben. Zudem umfasst die Vorrichtung ein Augenüberwachungsmodul zur Bestimmung von Augenbewegungen, und ist eingerichtet, das Positionierungsmodul basierend auf den bestimmten Augenbewegungen für einen entsprechenden Positionierungsausgleich anzusteuern. Da das Positionierungsmodul den Brennpunkt mit einer wesentlich kleineren Frequenz positioniert als das Abtastmodul, wirken sich Augenbewegungen auch entsprechend stärker auf die Positionierung des Brennpunkts an die Ausgangspunkte aus. Durch die Bestimmung der Augenbewegungen, z.B. auf der Basis von Iris- oder Venenmuster (auf der Sclera oder er Retina), kann der Einfluss der Augenbewegung jeweils bei der Positionierung des Brennpunkts an einen neuen Ausgangspunkt kompensiert werden. Die hohe Ablenkungsgeschwindigkeit des Abtastmoduls für die Erzeugung von Hohlräumen entsprechend dem Abtastmuster und die Kompensation von Augenbewegungen bei der Positionierung der Ausgangspunkte ermöglichen die refraktive Korrektur des Auges, ohne das Auge dafür am Lichtprojektor fixieren zu müssen. Durch die hohe Anzahl der Bearbeitungsteilgebiete, beispielsweise hundert, mitteln sich Bewegungsartefakte aus dem Abtragergebnis heraus. Die Erzeugung der Vielzahl von getrennten Hohlräumen mit hoher Ablenkungsgeschwindigkeit und die Kompensation der Augenbewegungen bei der Positionierung der Hohlräume ermöglichen somit die refraktive Korrektur des Auges mittels Laserpulsen, ohne dass das Auge und/oder der Patient in irgend einer Form mechanisch mit dem Lasersystem verbunden oder fixiert werden müssen.

Vorzugsweise definiert das Abtastmuster ein Bearbeitungsteilgebiet, dessen Durchmesser kleiner als die Dicke der Hornhaut ist. Wenn die Ausmasse des Bearbeitungsteilgebiets mit aufgelöstem Augengewebe kleiner sind, als die Dicke der Hornhaut, dann ist der an der Hornhautoberfläche ersichtliche Dickenverlust kleiner, als es der Höhe des aufgelösten Augengewebes entsprechen würde, insbesondere, wenn das Bearbeitungsteilgebiet mit dem aufgelösten Augengewebe von der Hornhautoberfläche entfernt ist (beispielsweise mehr als die Hälfte eines Fokusdurchmessers). Über die seitliche Ausdehnung (Durchmesser) und die Tiefenpositionierung eines Bearbeitungsteilgebiets kann dieser Effekt (der beschränkten Dickenreduktion an der Hornhautoberfläche) beeinflusst werden. Somit können durch Bearbeitungsteilgebiete, deren Durchmesser kleiner als die Dicke der Hornhaut ist, Brechkraftänderungen erzielt werden, die kleiner sind, als die der aufgelösten Gewebehöhe entsprechende Brechkraftänderung. Gemäss einer Faustformel für LASIK (Laser In Situ Keratomileusis) entspricht beispielsweise ein Hornhautabtrag von 12µm ungefähr einer Dioptrie. Durch die Auflösung von Augengewebe in kleinen, von der Hornhautoberfläche entfernten Bearbeitungsteilgebieten können somit Korrekturen der Brechkraft vorgenommen werden, die feiner sind, als dies durch eine ausgedehnte Gewebeauflösung der selben Höhe mittels den selben Laserpulsen möglich wäre.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
Figur 1 a zeigt ein Blockdiagramm, welches schematisch eine ophthalmologische Vorrichtung bei der Behandlung eines Auges mittels eines fokussierten gepulsten Laserstrahls darstellt.
Figur 1b zeigt eine Aufsicht einer durch die ophthalmologische Vorrichtung gemäss einem Abtastmuster bearbeiteten Bearbeitungsfläche.
Figur 2 zeigt ein Flussdiagram, welches den Ablauf bei der refraktiven Korrektur von Augengewebe durch die Gewebeauflösung in einer Vielzahl von voneinander getrennten Bearbeitungsteilgebieten illustriert.
Figur 3a zeigt einen Querschnitt durch ein Segment einer Augenhornhaut, in welcher zur refraktiven Korrektur Augengewebe in einer Vielzahl von voneinander getrennten Bearbeitungsteilgebieten aufgelöst wird.
Figur 3b zeigt eine Aufsicht einer Augenhornhaut, in welcher Gewebe zur refraktiven Korrektur in einer Vielzahl von sich nicht berührenden, in einem ringförmigen Cluster nebeneinander und übereinander liegend angeordneten Bearbeitungsteilgebieten aufgelöst wird.
Figur 3c zeigt einen weiteren Querschnitt durch das Segment der Augenhornhaut, in welcher die von voneinander getrennten Bearbeitungsteilgebiete in äquidistanten, gekrümmten Bearbeitungsflächen angeordnet sind.
Figur 4 zeigt in der Aufsicht die Überlappung der Fokusdurchmesser von mehreren nacheinander folgenden Laserpulsen.

### Wege zur Ausführung der Erfindung

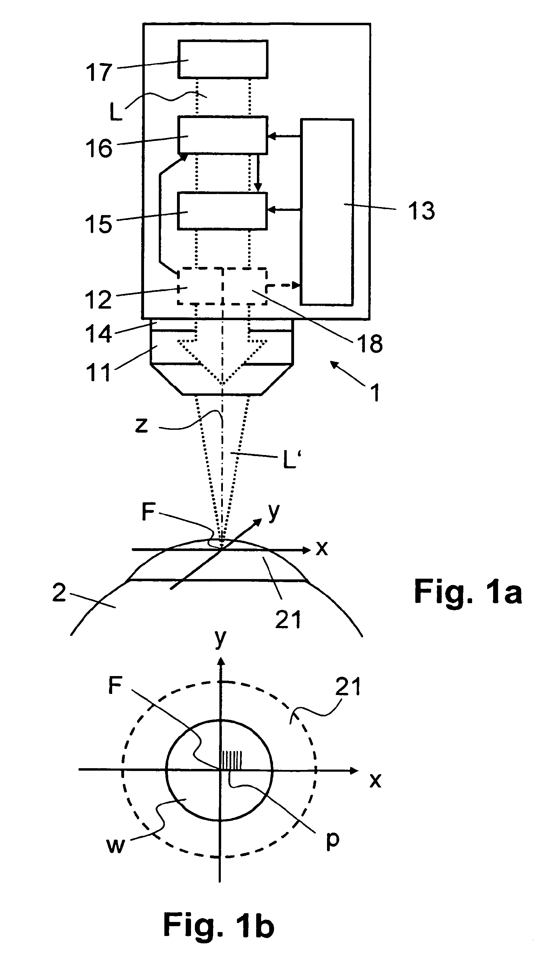

In der Figur 1a bezeichnet das Bezugszeichen 1 eine ophthalmologische Vorrichtung, respektive eine ophthalmologische Vorrichtungsanordnung, mit einer Laserquelle 17 und einem mit der Laserquelle 17 optisch verbundenen optischen Lichtprojektionsmodul 11 zur Erzeugung und fokussierten Projektion eines gepulsten Laserstrahls L' für die punktuelle Gewebeauflösung in einem Brennpunkt F (Fokus) im Innern des Augengewebes, beispielsweise in der Augenhornhaut 21 (Cornea). Die Laserquelle 17 umfasst insbesondere einen Femtosekundenlaser zur Erzeugung von Femtosekundenlaserpulsen, die Pulsbreiten von typisch 10fs bis 1000fs (1fs=10⁻¹⁵s) aufweisen. Die Laserquelle 17 ist in einem separaten oder in einem mit dem Lichtprojektionsmodul 11 gemeinsamen Gehäuse angeordnet.

Zum besseren Verständnis soll hier angeführt werden, dass die Figur 1a die ophthalmologische Vorrichtung 1 schematisch und vereinfacht darstellt. Zum Beispiel ist in der Figur 1a nicht präzise wiedergegeben, dass das optische Lichtprojektionsmodul 11 eine hohe numerische Apertur von mindestens 0.3 und vorzugsweise mehr als 0.4 aufweist, dass die ophthalmologische Vorrichtung 1 optional einen Saugring zur Befestigung am Auge 2 aufweist, oder dass die ophthalmologische Vorrichtung 1 optional einen Kontaktkörper (z.B. einen Applanationskörper) zur kontaktbasierten Verformung (z.B. zur Applanierung) des Auges 2 bei der Applikation der ophthalmologischen Vorrichtung 1 umfasst.

Wie in der Figur 1a schematisch dargestellt ist, umfasst die ophthalmologische Vorrichtung 1 ein Positionierungsmodul 16 und ein Abtastmodul 15, welche im schematisch dargestellten Strahlengang L zwischen Laserquelle 17 und Austritt des Lichtprojektionsmoduls 11 angeordnet sind. Der Fachmann wird verstehen, dass das Positionierungsmodul 16 und das Abtastmodul 15 auch in umgekehrter Reihenfolge angeordnet sein können, als dies in der Figur 1a dargestellt ist. Das Positionierungsmodul 16 und das Abtastmodul 15 sind kaskadierte Scannermodule, welche den Brennpunkt F positionieren respektive bewegen. Das Positionierungsmodul 16 ist ein wesentlich langsameres Scannermodul als das Abtastmodul 15.

Das Positionierungsmodul 16 ist eingerichtet, den Brennpunkt F an definierte Ausgangspunkte zu positionieren. Das Positionierungsmodul 16 umfasst beispielsweise Bewegungstreiber zum mechanischen Verschieben des Lichtprojektors 11 oder von Teilen des Lichtprojektors 11, zum Beispiel Bewegungstreiber für die laterale Verschiebung von Linsen. Die Bewegungstreiber umfassen beispielsweise ein Antriebselement für eine Vorschubrichtung x und ein Antriebselement für eine zur Vorschubrichtung x senkrechte Abtastrichtung y (siehe Figur 1b), beispielsweise Piezomotoren. In einer Ausführungsvariante umfasst das Positionierungsmodul 16 einen Galvanoscanner zum Ablenken der Laserpulse in der Vorschubrichtung x und/oder in der Abtastrichtung y. Die Koordinaten der Ausgangspunkte werden dem Positionierungsmodul 16 vorzugsweise vom Steuermodul 13 zugeführt, beispielsweise Punkt für Punkt oder als Datei mit (z.B. einer Sequenz von) mehreren Ausgangspunkten zum Speichern im Positionierungsmodul 16.

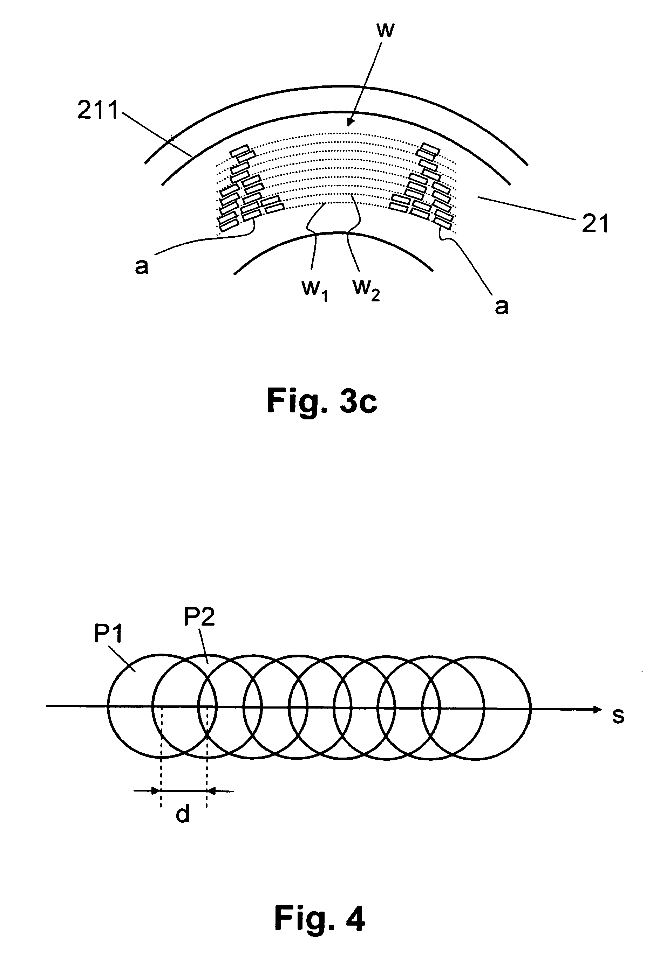

Das Abtastmodul 15 ist eingerichtet, den Brennpunkt F ausgehend vom aktuellen Ausgangspunkt gemäss einem definierten Abtastmuster p zu bewegen (siehe Figur 1b). Um die Laserpulse entsprechend dem vorgegebenen Abtastmuster p zu bewegen, umfasst das Abtastmodul 15 Ablenkungselemente, beispielsweise einen Galvanoscanner, einen resonanten Spiegelscanner, einen akustischen optischen Modulator (AOM), einen Polygonscanner oder einen mikroelektromechanischer Scanner (MEM). In einer Variante umfasst das Abtastmodul 15 zur Positionierung in einer gegenüber der Abtastrichtung y viel langsameren Vorschubrichtung x Bewegungstreiber zum mechanischen Verschieben des Lichtprojektors 11 oder von Teilen des Lichtprojektors 11, d.h. die Bewegung in der langsameren Vorschubrichtung x des Abtastmusters p kann beispielsweise durch das Positionierungsmodul 16 ausgeführt werden. Das Abtastmodul 15 ist entweder fest für ein bestimmtes Abtastmuster p oder für mehrere wählbare Abtastmuster p eingerichtet. Das Abtastmuster p definiert beispielsweise ein rechteckiges, rundes, elliptisches, sternförmiges, spiralförmiges oder Lissajou-Figur-förmiges Bearbeitungsteilgebiet a, und spezifiziert entsprechende Ablenkungen der Laserpulse in der Vorschubrichtung x und in der Abtastrichtung y. Zur refraktiven Korrektur der Augenhornhaut 21 definiert das Abtastmuster p vorzugsweise ein Bearbeitungsteilgebiet a, dessen Durchmesser kleiner als die Dicke der Augenhornhaut 21 ist. Vorzugsweise umfasst das Abtastmodul 15 die Ansteuerung der Ablenkungselemente zur Bewegung des Brennpunkts F gemäss dem Abtastmuster p, der Fachmann wird jedoch verstehen, dass die Ansteuerung auch durch das Steuermodul 13 erfolgen kann. Gegebenenfalls erfolgt die Auswahl eines Abtastmusters p durch das Steuermodul 13, beispielsweise durch entsprechende Auswahlinstruktionen oder Steuersequenzen für das betreffende Auswahlmuster p. Das Abtastmodul 15 ist zudem eingerichtet, die Laserpulse so Abzulenken, dass sich die Fokusdurchmesser P1, P2 von nacheinander folgenden Laserpulsen teilweise überlappen. Wie in der Figur 4 dargestellt ist überlappen sich die Fokusdurchmesser P1, P2 von in der Abtastrichtung s nacheinander folgenden Laserpulsen vorzugsweise um mehr als die Hälfte ihres Durchmessers, das heisst der Abstand d zwischen den Zentren der Fokusdurchmesser P1, P2 ist kleiner als der Radius der Fokusdurchmesser P1, P2. Das Abtastmodul 15 ist eingerichtet, den Brennpunkt F wesentlich schneller zu bewegen als sich das menschliche Auge bei einer Blickrichtungsänderung bewegt. Insbesondere ist das Abtastmodul 15 eingerichtet, die Laserpulse so schnell abzulenken, dass das Augengewebe in einem durch das Abtastmuster definierten Bearbeitungsteilgebiet a ohne darin verbleibende Gewebebrücken aufgelöst wird, auch wenn sich das Auge 2 bewegt. Das gesamte Abtastmuster p für ein Bearbeitungsteilgebiet wird durch das Abtastmodul 15 beispielsweise in 1 ms (Millisekunde) abgefahren. Wenn das Abtastmodul 15 zum Beispiel eingerichtet ist, ein Abtastmuster mit 20KHz in der Vorschubrichtung x abzufahren, können in 1 ms 20 Abtastzeilen in der Abtastrichtung y gefahren werden, bei einem Fokusdurchmesser beispielsweise im Bereich von 5µm bis 20µm wird das Augengewebe in einem Bearbeitungsteilgebiet a mit einem Durchmesser im Bereich von ungefähr 100µm bis 400µm aufgelöst (bei Überlappung der Fokusdurchmesser P1, P2 in der Vorschubrichtung x wird dieser Wert entsprechend reduziert). Eine eventuelle Augenbewegung in x Richtung wird diesen Bereich geringfügig strecken oder stauchen, wobei ein durchgehender Schnitt immer gewährleistet bleibt.

Eine ophthalmologische Vorrichtung mit einer mechanischen Bewegung des Lichtprojektors und einer Überlagerung einer zusätzlichen feinen Bewegung des Brennpunkts F mittels optischer Microscans wird in der per Referenz miteingeschlossenen EP 1 486 185 beschrieben. In der noch nicht veröffentlichten Europäischen Patentanmeldung Nr. 05 405 376 werden ein Scannermodul zur Ablenkung des gepulsten Lichtstrahls für die zusätzliche feine Bewegung, sowie ein optisches Übertragungssystem zur Übertragung der abgelenkten Femtosekundenlaserpulse vom Scannermodul zum Lichtprojektor 11 und zur Überlagerung der abgelenkten Femtosekundenlaserpulse auf die Bewegung des Lichtprojektors 11 beschrieben.

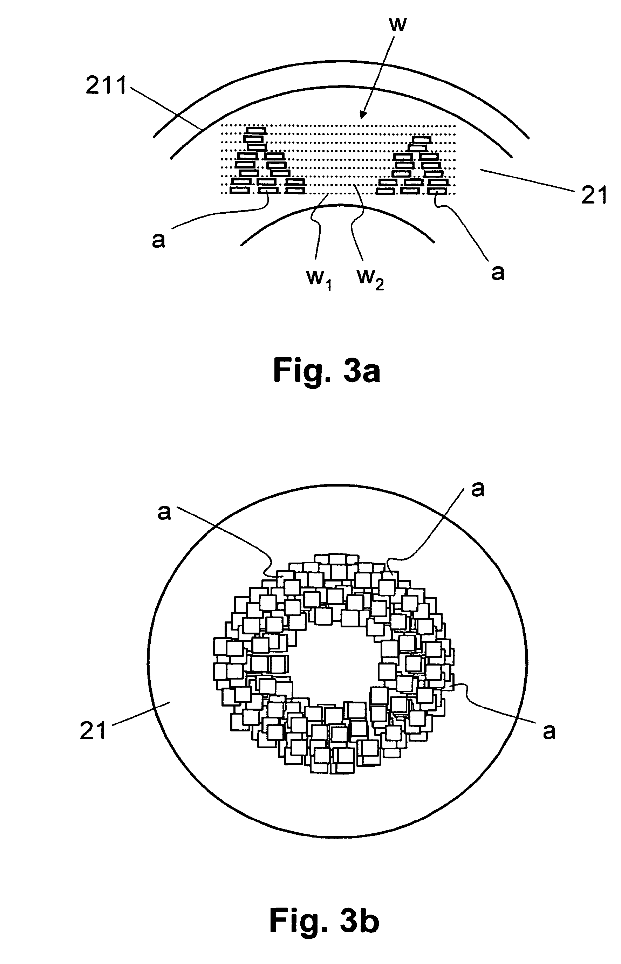

Wie in der Figur 1 a schematisch dargestellt ist, umfasst die ophthalmologische Vorrichtung 1 zudem ein Tiefenpositionierungsmodul 14 zum Verschieben des Brennpunkts F entlang einer Projektionsachse z des Lichtprojektors 11, beispielsweise senkrecht zu einer durch die Vorschubrichtung x und die Abtastrichtung y aufgespannten Bearbeitungsfläche, insbesondere eine Bearbeitungsebene w. Zum Verschieben des Brennpunkts F umfasst das Tiefenpositionierungsmodul 14 vorzugsweise eine bewegliche Fokussierungslinse und ein damit gekoppeltes Antriebselement. In einer alternativen Variante wird der Lichtprojektor 11 zur Tiefeneinstellung mechanisch bewegt. Wie im schematischen Querschnitt der Figur 3a illustriert wird, werden durch die Tiefeneinstellung des Brennpunkts F unterschiedlich tiefe Fokalebenen respektive Fokalflächen definiert, welche als Bearbeitungsebenen respektive Bearbeitungsflächen w, w₁, w₂ dienen, auf denen das Augengewebe, beispielsweise die Augenhornhaut 21, jeweils in mehreren voneinander getrennten, durch Abtastmuster p definierten Bearbeitungsteilgebieten a aufgelöst wird. Zusätzlich zu den in der Figur 3a dargestellten ebenen Bearbeitungsflächen w, w₁, w₂ können die Bearbeitungsflächen w, w₁, w₂ durch entsprechend gesteuerte Tiefeneinstellung des Brennpunkts F oder durch die Verwendung im Lichtprojektor 11 von Objektiven mit sphärischen Bildfeldern auch gekrümmt (konkav, konvex) ausgestaltet werden, wie dies in der Figur 3c dargestellt ist. Durch die Gewebeauflösung entsteht in jedem der Bearbeitungsteilgebiete a ein Hohlraum, der jeweils von den anderen Hohlräumen, sowohl auf der selben Bearbeitungsfläche w, w₁, w₂ als auch auf benachbarten, übereinander liegenden Bearbeitungsflächen w₁, w₂, durch Gewebebrücken getrennt ist. Vorzugsweise werden die Bearbeitungsteilgebiete a von der Hornhautoberfläche entfernt angeordnet, vorzugsweise unter der Bowmanschen Membran 211 der Hornhaut 21. Die Figur 3b illustriert in der Aufsicht ein Beispiel von einer Vielzahl von sich nicht berührenden Bearbeitungsteilgebieten a (beispielsweise hundert oder mehr), die in einem ringförmigen Cluster nebeneinander und übereinander liegend angeordnet sind, zur Bildung einer Vielzahl von voneinander getrennten, nicht zusammenhängenden Hohlräumen, welche kollabieren und dadurch die Hornhautkrümmung für eine gewünschte refraktive Korrektur der Augenhornhaut 21 geeignet ändern.

In einer weiteren Ausführungsvariante umfasst die ophthalmologische Vorrichtung 1 einen Wellenfrontdetektor 18 zum Bestimmen eines Wellenfrontverlaufs eines durch das Auge 2 reflektierten Lichtbündels. Das reflektierte Lichtbündel ist ein zusätzlicher Referenzlichtstrahl, der vom Augenhintergrund reflektiert und dem Wellenfrontdetektor 18 mittels optischer Elemente zugeführt wird. Der Wellenfrontdetektor ist beispielsweise als Shack-Hartmann-Sensor ausgeführt, beispielsweise nach US 2003/0038921, oder als Interferometer, z.B. als Shearing-Interferometer. Weitere mögliche Ausführungsformen des Wellenfrontdetektors sind in Jos. J. Rozena, Dirk E.M. Van Dyck, Marie-Jose Tassignon, "Clinical comparison of 6 aberrometers. Part 1: Technical specifications", J Cataract Refract Surg, Volume 31, Juni 2005, Seiten 1114-1127, beschrieben. Zur Rückmeldung des bestimmten Wellenfrontverlaufs ist der Wellenfrontdetektor 18 mit dem Steuermodul 13 verbunden. Das Steuermodul 13 ist eingerichtet, basierend auf dem bestimmten Wellenfrontverlauf die aktuell erreichte refraktive Korrektur der Augenhornhaut 21 zu bestimmen und darauf basierend die örtliche Verteilung weiterer Bearbeitungsteilgebiete a respektive die Ausgangspunkte für entsprechende Abtastmuster p zu bestimmen, um die gewünschte refraktive Korrektur der Augenhornhaut 21 zu erreichen. Je nach Ausführungsvariante wird die Bestimmung des Wellenfrontverlaufs und der Ausgangspunkte für weitere Bearbeitungsteilgebiete a zu unterschiedlichen Zeitpunkten durchgeführt, beispielsweise periodisch nach einem vorgegebenen Zeitplan, nach der Auflösung des Augengewebes in sämtlichen geplanten Bearbeitungsteilgebieten a auf einer Bearbeitungsfläche w, w₁, w₂, nach der Bearbeitung sämtlicher geplanter Bearbeitungsteilgebiete a und/oder nach dem Empfang eines über eine Benutzerschnittstelle eingegebenen Instruktionssignals.

In einer weiteren Ausführungsvariante umfasst die ophthalmologische Vorrichtung 1 ein Augenüberwachungsmodul 12 (ein so genannter "Eye Tracker") zur Bestimmung von Augenbewegungen. Das Augenüberwachungsmodul 12 umfasst beispielsweise eine Kamera, beispielsweise eine CCD-Kamera (Charged Coupled Device) und eine Beleuchtungseinrichtung (z.B. LEDs), zur Erfassung einer Aufsicht des Auges 2, sowie Verarbeitungsmittel zur Bestimmung des Iris- oder Venenmusters (auf der Sclera oder der Retina) in der Aufsicht und zur Bestimmung von Augenbewegungen basierend auf relativen Verschiebungen des Iris- oder Venenmusters. Die Verarbeitungsmittel sind als programmiertes Logikmodul mittels Software und/oder Hardware ausgeführt und sind in einer Variante im Steuermodul 13 angeordnet. Detektierte Augenbewegungen werden vom Augenüberwachungsmodul 12 laufend an das Positionierungsmodul 16 oder an das Steuermodul 13 übertragen, beispielsweise als Relativwerte bezüglich einer definierten Referenzposition des Auges 2 oder als Blickrichtungswerte des Auges 2. Das Positionierungsmodul 16 respektive das Steuermodul 13 ist eingerichtet, bei der Positionierung des Brennpunkts F in einen Ausgangspunkt Bewegungen des Auges 2 basierend auf den bestimmten Augenbewegungen auszugleichen. Das Positionierungsmodul 16 korrigiert die Koordinaten vorgegebener Ausgangspunkte basierend auf den detektierten Augenbewegungen oder das Steuermodul 13 liefert dem Positionierungsmodul 16 Ausgangspunkte, deren Koordinaten entsprechend den Augenbewegungen angepasst sind.

Das Steuermodul 13 ist vorzugsweise als programmiertes Logikmodul mittels Software und/oder Hardware ausgeführt. Das Steuermodul 13 ist zur Übertragung von Steuersignalen und/oder Steuerdaten mit dem Positionierungsmodul 16 und dem Abtastmodul 15 verbunden. Je nach Ausführungsvariante ist das Steuermodul 13 zum Empfang von Rückmeldungen oder Datenwerten über Augenbewegungen mit dem Wellenfrontdetektor 18 und/oder dem Augenüberwachungsmodul 12 verbunden. Das Steuermodul 13 ist in einem separaten oder in einem mit dem Lichtprojektionsmodul 11 gemeinsamen Gehäuse angeordnet. Das Steuermodul 13 ist eingerichtet, für eine gewünschte refraktive Korrektur des Auges 2, insbesondere der Augenhornhaut 21, die örtliche Verteilung der dazu nötigen Bearbeitungsteilgebiete a im Innern des Auges 2 zu bestimmen, das heisst die Anzahl der Bearbeitungsteilgebiete a, die jeweils zugeordneten Ausgangspunkte (in mehreren Bearbeitungsflächen) und in einer Variante auch das entsprechende Abtastmuster p respektive die durch das Abtastmuster p definierte Grösse, Form und/oder Ausrichtung der Bearbeitungsteilgebiete a. In einer Variante ist das Steuermodul 13 eingerichtet, den Wellenfrontverlauf eines durch das Auge 2 reflektierten Lichtbündels und damit die aktuelle Brechkraft der Augenhornhaut 21 mittels des Wellenfrontdetektors 18 zu ermitteln, und darauf basierend die örtliche Verteilung der Bearbeitungsteilgebiete a im Innern des Auges 2 zu bestimmen.

Das Steuermodul 13 bestimmt die Anzahl und örtliche Verteilung der Bearbeitungsteilgebiete a beispielsweise auf Grund einer Tabelle. Die Tabelle ordnet verschiedenen refraktiven Korrekturwerten (und Korrekturarten) jeweils eine Anzahl und örtliche Verteilung der Bearbeitungsteilgebiete a zu. In einer weiteren Variante bestimmt das Steuermodul 13 die Anzahl und örtliche Verteilung der Bearbeitungsteilgebiete a auf Grund eines Modells des zu behandelnden Augengewebes, z.B. ein Augenhornhautmodel und Informationen darüber, wie das Auge 2 abbildet, bei vorgegebener Grösse und Form der Bearbeitungsteilgebiete a und bei vorgegebenen vertikalen und horizontalen Mindestabständen einzelner Bearbeitungsteilgebiete a. Die Angaben über die Anzahl und die örtliche Verteilung der Bearbeitungsteilgebiete a können auch von einer externen Einheit an das Steuermodul 13 übermittelt werden. Die örtliche Verteilung der Bearbeitungsteilgebiete a erfolgt so, dass die bei der Auflösung des Augengewebes in den Bearbeitungsteilgebieten a entstehenden Hohlräume sowohl auf der selben Bearbeitungsfläche w, w₁, w₂ als auch in benachbarten, übereinander liegenden Bearbeitungsflächen w₁, w₂ jeweils durch Gewebebrücken voneinander getrennt sind. Die Anzahl der Bearbeitungsteilgebiete a kann in einer Variante auch aus einem Abtragsvolumen bestimmt werden, dass für eine spezifizierte refraktive Korrektur bestimmt oder durch den Benutzer eingegeben wird. Die örtliche Anordnung der Bearbeitungsteilgebiete a wird durch die Korrekturart bestimmt, beispielsweise muss bei einer Myopie die Augenhornhaut 21 durch zentralisierte Abtragung abgeflacht werden, wohingegen bei einer Hyperopie die Krümmung der Hornhautoberfläche durch ringförmig umlaufende Abtragung steiler ausgestaltet werden muss.

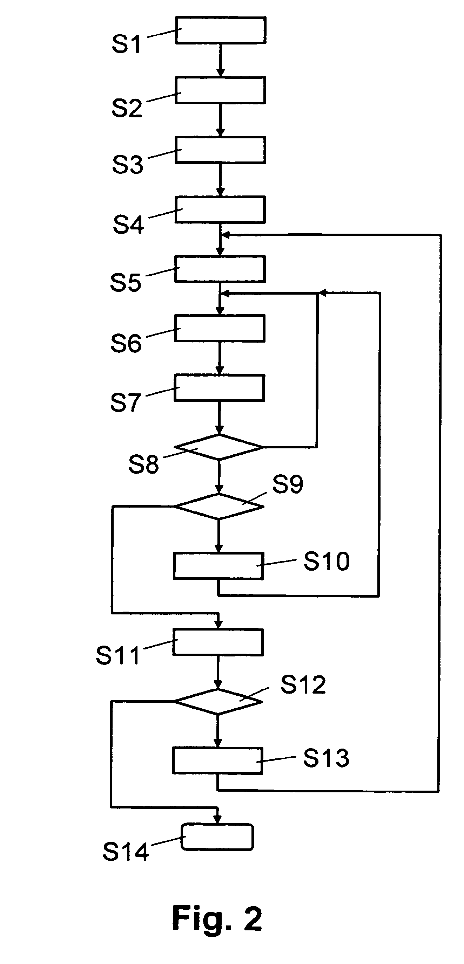

In den nachfolgenden Abschnitten wird mit Bezug zu der Figur 2 der durch das Steuermodul 13 gesteuerte Ablauf bei der refraktiven Korrektur eines Auges 2 beschrieben.

Im Schritt S1 bestimmt das Steuermodul 13 die gewünschte refraktive Korrektur des Auges 2. Der Sollwert der refraktiven Korrektur wird beispielsweise über eine Benutzerschnittstelle eingegeben und im Steuermodul 13 erfasst.

Im Schritt S2 bestimmt das Steuermodul 13 die örtliche Verteilung der Bearbeitungsteilgebiete a, um die gewünschte refraktive Korrektur zu erreichen.

Im optionalen Schritt S3 übermittelt das Steuermodul 13 die Ausgangspunkte für die Abtastmuster p der Bearbeitungsteilgebiete a an das Positionierungsmodul 16 beispielsweise als Sequenz von Ausgangspunkten, geordnet nach abnehmender Tiefe der Bearbeitungsfläche w, w₁, w₂. In einer Variante sind den verschiedenen Ausgangspunkten auch Identifizierungselemente von unterschiedlichen Abtastmustern p zugeordnet. Gegebenenfalls werden auch Steuerwerte für unterschiedliche Abtastmuster p an das Abtastmodul 15 übermittelt.

Im Schritt S4 wird die Behandlung des Auges 2 durch ein über die Benutzerschnittstelle eingegebenes Startsignal gestartet.

Im Schritt S5 wird der Brennpunkt F auf die tiefstgelegene Bearbeitungsfläche w₁ positioniert. Die entsprechende Ansteuerung des Tiefenpositionierungsmoduls 14 erfolgt vorzugsweise durch das Steuermodul 13.

Im Schritt S6 positioniert das Positionierungsmodul 16 den Brennpunkt F in der aktuellen Bearbeitungsfläche w, w₁, w₂ an einen noch nicht verwendeten Ausgangspunkt. Die Positionierung erfolgt gemäss Vorgabe des Ausgangspunkt durch das Steuermodul 13 oder gemäss einer vorher im Positionierungsmodul 16 gespeicherten Sequenz von Ausgangspunkten. Bei der Positionierung werden auch die dauernd überwachten Augenbewegungen berücksichtigt und entweder im Steuermodul 13 oder im Positionierungsmodul 16 kompensiert.

Im Schritt S7 bewegt das Abtastmodul 15 den Brennpunkt F in der aktuellen Bearbeitungsfläche w, w₁, w₂, ausgehend vom aktuellen Ausgangspunkt entsprechend dem Abtastmuster p, das dem aktuellen Ausgangspunkt zugeordnet ist. Das zu verwendende Abtastmuster p ist beispielsweise für die gesamte Behandlung unverändert oder wird durch das Steuermodul 13 oder das Positionierungsmodul 16, beispielsweise bei der Übermittlung eines Synchronisationssignals, mittels eines Identifizierungselements bestimmt.

Im Schritt S8 überprüft das Steuermodul 13, ob sämtliche Ausgangspunkte der aktuellen Bearbeitungsfläche w, w₁, w₂ bereits bearbeitet wurden. Falls zu bearbeitende Ausgangspunkte verbleiben, erfolgt die Positionierung des nächsten Ausgangspunkt im Schritt S6. Falls auf der aktuellen Bearbeitungsfläche w, w₁, w₂ alle zugeordneten Ausgangspunkte bearbeitet wurden, fährt das Steuermodul im Schritt S9 fort.

Im Schritt S9 überprüft das Steuermodul 13, ob sämtliche Bearbeitungsflächen w, w₁, w₂ bereits bearbeitet wurden. Falls zu bearbeitende Bearbeitungsflächen w, w₁, w₂ verbleiben, wird der Brennpunkt F im Schritt S10 auf die nächst höher gelegene, äquidistante (z.B. parallele) Bearbeitungsfläche w, w₂ positioniert, auf der Ausgangspunkte zu bearbeiten sind, und das Positionierungsmodul 16 fährt im Schritt S6 mit der Positionierung des nächsten Ausgangspunkts fort. Ansonsten, wenn sämtliche Bearbeitungsflächen w, w₁, w₂ mit zu bearbeitenden Ausgangspunkten bereits bearbeitet wurden, fährt das Steuermodul im Schritt S11 fort.

Im optionalen Schritt S11 bestimmt der Wellenfrontdetektor 18 den Wellenfrontverlauf des Auges 2 und übermittelt diesen an das Steuermodul 13 (ohne Schritte S11, S12, S13 endet das Verfahren im Schritt S14).

Im Schritt S12 bestimmt das Steuermodul 13 auf Grund des Wellenfrontverlaufs, ob die gewünschte refraktive Korrektur erreicht wurde. Falls die gewünschte Korrektur erreicht wurde, beendet das Steuermodul 13 das Verfahren im Schritt S14, beispielsweise mit einer Erfolgsmeldung über die Benutzerschnittstelle. Andernfalls, wenn die gewünschte refraktive Korrektur noch nicht erreicht wurde, fährt das Steuermodul im Schritt S13 fort.

Im Schritt S13 bestimmt das Steuermodul 13, vorzugsweise nach erfolgter Rückmeldung und Bestätigung über die Benutzerschnittstelle, die örtliche Verteilung zusätzlicher Bearbeitungsteilgebiete a, die zum Erreichen der gewünschten refraktiven Korrektur zu bearbeiten sind. Die Bearbeitung der weiteren Bearbeitungsteilgebiete a erfolgt im Schritt S5, gegebenenfalls nach der Übermittlung der zusätzlichen Ausgangspunkte an das Positionierungsmodul 16.

## Patentansprüche

1. Ophthalmologische Vorrichtung (1) für eine refraktive Korrektur eines Auges (2), umfassend:
eine laserqüelle (17),
einen Lichtprojektor (11) zur Projektion von Laserpulsen (L') auf einen Brennpunkt (F) im Innern des Auges (2) für eine Auflösung von Augengewebe, und
ein Positionierungsmodul (16) und ein Abtastmodul (15), welche als kaskadierte Scannermodule zum Ablenken der Laserpulse (L') im Strahlengang (L) zwischen Laserquelle (17) und Austritt des Lichtprojektors (11) angeordnet sind, wobei das Abtastmodul (15) eingerichtet ist, den Brennpunkt (F) wesentlich schneller zu bewegen als eine Bewegungsgeschwindigkeit eines menschlichen Auges (2) bei einer Blickrichtungsänderung, **dadurch gekennzeichnet,**
**dass** das Positionierungsmodul (16) ein wesentlich langsameres Scannermodul als das Abtastmodul (15) ist und einen im Strahlengang (L) nach dem Abtastmodul (15) angeordneten Galvanoscanner zum Ablenken der Laserpulse (L') in einer Vorschubrichtung (x) und in einer zur Vorschubrichtung (x) senkrechten Abtastrichtung (y) umfasst.

2. Vorrichtung (1) nach Anspruch 1, **dadurch gekennzeichnet, dass** die Vorrichtung (1) ein Augenüberwachungsmodul (12) zur Bestimmung von Augenbewegungen umfasst.

3. Vorrichtung (1) nach Anspruch 2, **dadurch gekennzeichnet, dass** das Positionierungsmodul (16) eingerichtet ist, den Brennpunkt (F) an unterschiedliche Ausgangspunkte im Innern der Hornhaut (21) zu positionieren und dabei Bewegungen des Auges (2) basierend auf den bestimmten Augenbewegungen auszugleichen.

4. Vorrichtung (1) nach Anspruch 3, **dadurch gekennzeichnet, dass** das Abtastmodul (15) eingerichtet ist, den Brennpunkt (F) ausgehend von jeweils einem der Ausgangspunkte gemäss einem Abtastmuster (p) zu bewegen um ein Bearbeitungsteilgebiet (a) im Innern der Hornhaut (21) mit jeweils mehreren Laserpulsen (L') zu bearbeiten.

5. Vorrichtung (1) nach einem der Ansprüche 1 bis 4, **dadurch gekennzeichnet, dass** das Abtastmodul (15) zum Ablenken der Laserpulse (L') einen Galvanoscanner umfasst.

6. Vorrichtung (1) nach einem der Ansprüche 1 bis 5, **dadurch gekennzeichnet, dass** das Abtastmodul (15) zum Ablenken der Laserpulse (L') einen resonanten Spiegelscanner umfasst.

7. Vorrichtung (1) nach einem der Ansprüche 1 bis 6, **dadurch gekennzeichnet, dass** das Abtastmodul (15) zum Ablenken der Laserpulse (L') einen akustischen optischen Modulator umfasst.

8. Vorrichtung (1) nach einem der Ansprüche 1 bis 7, **dadurch gekennzeichnet, dass** das Abtastmodul (15) zum Ablenken der Laserpulse (L') einen Polygonscanner umfasst.

9. Vorrichtung (1) nach einem der Ansprüche 1 bis 8, **dadurch gekennzeichnet, dass** das Abtastmodul (15) zum Ablenken der Laserpulse (L') einen mikroelektromechanischen Scanner umfasst.

10. Vorrichtung (1) nach einem der Ansprüche 1 bis 9, **dadurch gekennzeichnet, dass** das Abtastmodul (15) eingerichtet ist, nacheinander folgende Laserpulse (L') so zu positionieren, dass sich ihre Fokusdurchmesser (P1, P2) teilweise überlappen.

11. Vorrichtung (1) nach Anspruch 10, **dadurch gekennzeichnet, dass** sich ihre Fokusdurchmesser (P1, P2) mindestens bis zur Hälfte ihres Durchmessers überlappen.

12. Vorrichtung (1) nach einem der Ansprüche 1 bis 11, **gekennzeichnet durch** ein Tiefenpositionierungsmodul (14) zum Verschieben des Brennpunkts (F) entlang einer Projektionsachse (z) des Lichtprojektors (11).

## Claims

1. Ophthalmological device (1) for refractive correction of an eye (2), comprising:
a laser source (17),
a light projector (11) for projecting laser pulses (L') on a focus (F) in the interior of the eye (2) for breaking up eye tissue, and
a positioning module (16) and a scanning module (15), which are arranged between laser source (17) and outlet of the light projector (11) as cascaded scanner modules for deflecting the laser pulses (L') in the beam path (L), the scanning module (15) being designed to move the focus (F) substantially faster than a movement speed of a human eye (2) when changing the direction of view, **characterized**
**in that** the positioning module (16) is a substantially slower scanner module than the scanning module (15) and comprises a galvano scanner, arranged in the beam path (L) downstream of the scanning module (15), for deflecting the laser pulses (L') in an advance direction (x) and in a scanning direction (y) perpendicular to the advance direction (x).

2. Device (1) according to Claim 1, **characterized in that** the device (1) comprises an eye monitoring module (12) for determining eye movements.

3. Device (1) according to Claim 2, **characterized in that** the positioning module (16) is designed to position the focus (F) at different initial points in the interior of the cornea (21) and, in the process, compensate for movements of the eye (2) on the basis of the determined eye movements.

4. Device (1) according to Claim 3, **characterized in that** the scanning module (15) is designed to move the focus (F) from respectively one of the initial points as per a scanning pattern (p) in order to treat a treatment subsection (a) in the interior of the cornea (21), respectively using a plurality of laser pulses (L').

5. Device (1) according to one of Claims 1 to 4, **characterized in that** the scanning module (15) for deflecting the laser pulses (L') comprises a galvano scanner.

6. Device (1) according to one of Claims 1 to 5, **characterized in that** the scanning module (15) for deflecting the laser pulses (L') comprises a resonant mirror scanner.

7. Device (1) according to one of Claims 1 to 6, **characterized in that** the scanning module (15) for deflecting the laser pulses (L') comprises an acousto-optic modulator.

8. Device (1) according to one of Claims 1 to 7, **characterized in that** the scanning module (15) for deflecting the laser pulses (L') comprises a polygon scanner.

9. Device (1) according to one of Claims 1 to 8, **characterized in that** the scanning module (15) for deflecting the laser pulses (L') comprises a microelectromechanical scanner.

10. Device (1) according to one of Claims 1 to 9, **characterized in that** the scanning module (15) is designed to position successive laser pulses (L') such that the focal diameters (P1, P2) thereof partly overlap.

11. Device (1) according to Claim 10, **characterized in that** their focal diameters (P1, P2) at least overlap up to half of the diameter thereof.

12. Device (1) according to one of Claims 1 to 11, **characterized by** a depth positioning module (14) for displacing the focus (F) along a projection axis (z) of the light projector (11).

## Revendications

1. Dispositif ophtalmologique (1) pour une correction réfractive d'un oeil (2), comprenant :
une source laser (17),
un projecteur de lumière (11) pour projeter des impulsions laser (L') sur un foyer (F) à l'intérieur de l'oeil (2) pour une désagrégation du tissu oculaire, et
un module de positionnement (16) et un module de palpage (15) qui sont disposés dans le trajet du rayon (L) entre la source laser (17) et la sortie du projecteur de lumière (11) sous la forme de modules scanners montés en cascade pour dévier l'impulsion laser (L'), le module de palpage (15) étant configuré pour déplacer le foyer (F) considérablement plus vite qu'une vitesse de mouvement d'un oeil humain (2) lors d'un changement de direction d'observation, **caractérisé en ce que** le module de positionnement (16) est un module scanner considérablement plus lent que le module de palpage (15) et comprend un galvanoscanner disposé dans le trajet du rayon (L) après le module de palpage (15) pour dévier l'impulsion laser (L') dans une direction d'avance (x) et dans une direction de palpage (y) perpendiculaire à la direction d'avance (x).

2. Dispositif (1) selon la revendication 1, **caractérisé en ce que** le dispositif (1) comprend un module de surveillance de l'oeil (12) pour déterminer les mouvements de l'oeil.

3. Dispositif (1) selon la revendication 2, **caractérisé en ce que** le module de positionnement (16) est configuré pour positionner le foyer (F) en différents points de départ à l'intérieur de la cornée (21) et pour compenser ainsi les mouvements de l'oeil (2) en se basant sur les mouvements déterminés de l'oeil.

4. Dispositif (1) selon la revendication 3, **caractérisé en ce que** le module de palpage (15) est configuré pour déplacer le foyer (F) selon un modèle de palpage (p) à chaque fois à partir de l'un des points de départ afin de traiter une région partielle de traitement (a) à l'intérieur de la cornée (21) à chaque fois avec plusieurs impulsions laser (L').

5. Dispositif (1) selon l'une des revendications 1 à 4, **caractérisé en ce que** le module de palpage (15) comprend un galvanoscanner pour la déviation des impulsions laser (L').

6. Dispositif (1) selon l'une des revendications 1 à 5, **caractérisé en ce que** le module de palpage (15) comprend un scanner à miroir résonnant pour la déviation des impulsions laser (L').

7. Dispositif (1) selon l'une des revendications 1 à 6, **caractérisé en ce que** le module de palpage (15) comprend un modulateur opto-acoustique pour la déviation des impulsions laser (L').

8. Dispositif (1) selon l'une des revendications 1 à 7, **caractérisé en ce que** le module de palpage (15) comprend un scanner polygone pour la déviation des impulsions laser (L').

9. Dispositif (1) selon l'une des revendications 1 à 8, **caractérisé en ce que** le module de palpage (15) comprend un scanner microélectromécanique pour la déviation des impulsions laser (L').

10. Dispositif (1) selon l'une des revendications 1 à 9, **caractérisé en ce que** le module de palpage (15) est configuré pour positionner les impulsions laser (L') qui se suivent successivement, de telle sorte que ses diamètres de foyer (P1, P2) se chevauchent partiellement.

11. Dispositif (1) selon la revendication 10, **caractérisé en ce que** ses diamètres de foyer (P1, P2) se chevauchent au moins jusqu'à la moitié de son diamètre.

12. Dispositif (1) selon l'une des revendications 1 à 11, **caractérisé par** un module de positionnement en profondeur (14) pour décaler le foyer (F) le long d'un axe de projection (z) du projecteur de lumière (11).
